# EUROPÄISCHE PATENTSCHRIFT

(11) **EP 0 981 310 B1**
(45) Veröffentlichungstag und Bekanntmachung des Hinweises auf die Patenterteilung: **25.09.2002**
(21) Anmeldenummer: 98928161.3
(22) Anmeldetag: 04.05.1998
(51) Int. Cl.: A61F 5/058

(54) **FRAKTUR-ROLL-ORTHESE**
SWIVELLING FRACTURE ORTHOSIS
ORTHESE PIVOTANTE POUR FRACTURE

(30) Priorität: 07.05.1997 DE 19719140
(43) Veröffentlichungstag der Anmeldung: 01.03.2000
(73) Patentinhaber: Gottsmann, Gert, 81375 München (DE)
(72) Erfinder: GOTTSMANN, Gert, D-81375 München (DE); PRALL, Paul, D-85238 Petershausen (DE); PETRA, Elke, D-85646 Anzing (DE)
(74) Vertreter: Petra, Elke, Dipl.-Ing.
(86) Internationale Anmeldenummer: DE9801231
(87) Internationale Veröffentlichungsnummer: WO98049979

(56) Entgegenhaltungen:
- EP-A- 0 190 543
- DE-C- 207 893
- GB-A- 2 108 849
- US-A- 1 389 525
- US-A- 2 667 868
- US-A- 3 232 289
- US-A- 4 576 153

## Beschreibung

Die Erfindung bezieht sich auf eine Fraktur-Orthese, insbesondere eine Humerus-Fraktur-Orthese (Oberarm-Ortbese) gemäß Oberbegriff des Patentanspruchs 1 bzw. 5, wie sie z.B. nach einer Fraktur zwischen proximalem und distalem Drittel des Humerusschaftes, entweder am Tag der Verletzung oder als Nachfolgebehandlung, nachdem der ursprüngliche Immobilisationsverband entfernt wurde, angelegt werden.

Solcherart Orthesen sind z.B. aus dem Prospekt 2/90 "MANUAL für die funktionelle konservative Frakturbehandlung mit dem Miami-Fraktur-Orthesen-System nach Sarmiento Latta" der Firma Thämert, 30938 Burgwedel, beschrieben. Die dort dargestellte und beschriebene Humerus-Fraktur-Orthese, System Miami, besteht aus zwei rinnenförmigen Halbschalen, die den Oberarm zwischen Achsel und Ellbogen umfassen und die über Klettverschlußbänder in Verbindung mit Kunststoffschlaufen zusammengehalten werden. Diese Halbschalen sind aus gelochtem glatten wasserfestem Kunststoff gefertigt und werden über einen doppelten Bauniwollstrumpf, der vorhergehend vom Ellbogen bis zur Achsel überangezogen wird, angelegt. Beim Anlegen wird zuerst die vordere Schale, danach die hintere Schale auf- bzw. angelegt, wonach zwei an der einen Längsseite zueinander beabstandet angebrachte Klettverschlußbänder durch Kunststoffschlaufen der Vorderschale hindurchgeführt, danach durch an der zweiten Längsseite der hinteren Schale entsprechend beabstandet befestigte weitere Kunststoffschlaufen hindurchgesteckt und um 180° wieder zurückgeführt und festgeklettet werden. Es erfolgt somit ein einseitiges Festzurren der Bänder, wodurch, abgesehen vom schmerzhaften Ziehen auch noch ein Verschieben der Orthese verursacht werden kann. Zudem verschiebt sich die so befestigte Orthese in ihrer Längsrichtung auf den darunter befindlichen Baumwollstrumpf, also sie rutscht nach unten, wodurch sich der Sitz lockert und zudem eine Behinderung in der Ellbogenneige stattfindet. Die Orthese muß dann immer wieder aufgemacht und in die richtige Position hochgeschoben und die Bänder wieder festgezogen werden, was einerseits schmerzhaft sein kann und andererseits sich auf die Fraktur negativ auswirkt. Andererseits ist durch die bekannte Orthese keine Kontrolle über die Bewegung des Oberarms gegeben. Ein vorhergehend, gleich nach der Fraktur angelegter Gipsverband (Immobilisationsverband) besitzt nämlich eine Kappe, die über die Schulter führt. Stößt der Patient bei der Bewegung seines Armes an die Kappe an, dann weiß er, daß er sofort den Arm hinunterziehen muß.

Dabei ist zu beachten, daß die Humerus-Fraktur-Orthesen, die in den unfallchirurgischen Abteilungen und Kliniken bei nicht operativen Frakturbehandlungen immer mehr eingesetzt werden, jeweils in drei oder vier Größen (S, M, L und XL) und zwar jeweils für den linken und den rechten Arm hergestellt und bereitgehalten werden müssen. Insbesondere die Lagerhaltung der in zusasaiengesetztem Zustand voluminösen Schalen-Hohlkörper ist sehr platzintensiv, wenn man bedenkt, daß insgesamt acht Ausführungsformen (vier Größen, jeweils für rechts und links) bevorratet werden müssen.

Zwar ist in diesem Prospekt auch eine Tibia-Fraktur-Orthese (Unterschenkel-Orthese) aufgezeigt, bei der eine Fersenkappe zur Positionsfestlegung der Tibia-Orthese stattfindet. Die Fersenkappe wird, nach vorhergehender Positionsbestimmung, über nicht wieder lösbare Haftstreifen an der hinteren Orthesen-Schale befestigt. Ist diese Befestigung nicht ordnungsgemäß erfolgt, kann ein Nacheinstellen in bezug auf Orthesenhöhe oder Kappenwinkel nicht mehr vorgenommen werden.

Aus der DE-PS 20 78 93 ist ein biegsames Holzbrettchen für Verbände, insbesondere zum Bandagieren von Knochenbrüchen bekannt, bei dem entweder schmale Holzleisten direkt nebeneinander auf Leinen o.dgl. aufgeleimt sind oder ein auf Leinen o.dgl. aufgeklebtes Brettchen außenseitig parallele Nuten aufweist, so daß diese zumindest in einer Richtung zusammenrollbar ist. Wie diese Einrichtung jedoch an den Gliedmaßen befestigt wird, ist der Druckschrift nicht zu entnehmen. Es darf angenommen werden, daß dies durch Bandagieren geschehen ist. Als selbstanlegbare Rollorthesen ist diese bekannte Einrichtung nicht verwendbar.

Schließlich ist aus der GB 2 108 849 A eine Beinschiene bekannt, bei der in relativ breiten Längstaschen als Längsversteifung Bretter eingeschoben sind. Diese Beinschiene wird durch mehrere, relativ kurze Bänder, mit Klettverschlußelementen an Bändern und Schienentaschen, zusammengehalten. Diese bekannte Einrichtung ist nur zur vorübergehenden Ruhigsetzung des Beines geeignet und kann keinesfalls z.B. als Oberarmorthese verwendet werden, die bei Nichtverwendung klein zusammenrollbar ist.

Aufgabe der Erfindung ist somit, eine Fraktur-Ortnese, insbesondere Humerus-Fraktur-Orthese obiger Gattung anzugeben, durch die es möglich ist, bei einfachem Aufbau, ein sicheres, atmungsaktives Ruhigstellen der Gleidteile zu ermöglichen, durch nur zwei alle Größen abdeckende, eng zusammenrollbare Orthesenausführungen (links und rechts) und die zudem einfach, positionsgenau und unverschieblich angelegt und getragen werden kann, mit optimaler Positions-Nacheinstellung.

Diese Aufgabe wird durch eine Fraktur-Orthese oben genannter Gattung mit den kennzeichnenden Merkmalen des Patentanspruchs 1 bzw. 5 gelöst. Weitere vorteilhafte Ausgestaltungen sind in den Unteransprüchen gekennzeichnet.

Demgemäß ist aus einer in Umfangsrichtung rollflexiblen Schale gebildete Orthese aus mehreren in Längserstreckung parallel zueinander angeordneten, die jeweils benachbart schwenkbar miteinander verbundenen biegesteifen Streifen zusammengesetzt und wird über Klettverschluß-bänder zusammengehalten. Dabei ist lediglich an der Innenseite der Streifen ein atmungaktives, hautfreudndliches Material wie Baumwollgewebe z.B. durch Kleben aufgebracht und die Klettverschluß-Bänder sind komplett über den äußeren Umfang der überlappend angelegten Schale immer in gleicher Anlege- oder Zugrichtung, hinweggeführt. Zudem sind die Streifen untereinander direkt gelenkig verbunden, wobei jeder Streifen an seinen beiden Längs-Schmalseiten eine seitlich herausragende, zumindestteilweise zylindrische, in Längsrichtung sich erstreckende Gelenkachse aufweist, die schwenkbar in eine sich in Längsrichtung erstreckende zylindrische Gelenkpfanne oder Lager der anderen Längs-Sehmalseite eines benachbarten Streifens längsverschiebbar und verschwenkbar, jedoch in Umfangsrichtung nicht herausziehbar, hereinragt.

Hierdurch wird eine Rollschale, also eine flexible "Roll-Humerus-Fraktur-Orthese" bereitgestellt, die in Längsrichtung biegesteif und in Querrichtung eng zusammenrollbar ist. In abgelegtem Zustand kann diese Rollschale flach auf eine Unterlage z.B. einen Tisch aufgelegt und/oder zum Verpacken in eine Schachtel oder zum Wegräumen in irgendein Behältnis, eng in Form einer Rolle zusammengedreht werden, wodurch eine sehr große Platzersparnis erreicht wird. Dadurch daß die Rollschale sich jeder Armdicke mühelos anpaßt bzw. sich an jedem noch so kleinen und noch so dicken Arm einfach anlegen läßt und nur an seinen beiden Längsenden dann mehr oder weniger überlappt, besteht die Möglichkeit, die erfindungsgemäße Orthese nur in einer einzigen Größe, d.h. in nur zwei Ausführungen, also Ausführung links und Ausführung rechts, zur Verfügung zu stellen.

Statt bisher acht Ausführungen reichen nunmehr zwei Ausführungen. Es sind somit die großen Vorteile der Platz- und Stückzahleinsparung, insbesondere hinsichtlich Lagerhaltung und Fertigung optimal erreicht. Auch das Anlegen der erfindungsgemäßen Roll-Schalen-Orthese ist denkbar einfach, da diese im wesentlichen über ihre Mitte längsseitig am Arm angelegt und die beiden Enden um den Arm sich überlappend fest umgelegt werden müssen, wonach über Klettverschluß-Bänder, vorzugsweise zwei Bänder, die Festlegung der Rollschale am Arm erfolgt. Zudem wird, dadurch daß die Klettverschluß-Bänder komplett um den äußeren Umfang der angelegten Roll-Schale hinweggeführt wird, immer in gleicher Anlege- bzw. Zugrichtung, ohne Richtungsänderung, ein sanftes Anlegen und Fixieren, ohne ruckartiges Zurückziehen bzw. Festzurren durch die Bänder erfolgen. Durch im Umfangsverlauf vorgesehen, mindestens eine Metall- oder Kunststoffschlaufe und ein oder mehrere Klettpunkte wird in vorteilhafter Weiterbildung eine noch größere Anlege- und Tragesicherheit erreicht. Statt der relativ aufwendigen Führungs-Schlaufen können auch lediglich ein oder mehrere Umfangwülste, Noppen o.ä. jeweils paarweise angeordnet sein, die. eine Positionsfestlegung bzw. -führung der Bänder auf dem Schalenumfang erlauben.

Die Gelenkelemente, also Achsenteil bzw. Gelenkschalen- oder Gelenkpfannenteil können unterschiedlich ausgebildet sein, wobei jede einschlägige bekannte Bauweise, insbesondere aus Rollläden, Rolljalousien, Rollschränken usw. bekannten Gelenkverbindungen zwischen den entsprechenden Streifen, Stäben usw., angewendet werden können. Immer ersteckt sich die im wesentlichen zylindrische Gelenkpfanne und auch die in diese schwenkbar eingreifende Gelenkachse entlang der gesamten Längserstreckung des Streifens bzw. der Leiste, wobei die Gelenkachse, je nach Festigkeitsbedarf ganzstückig oder in mehrere, zueinander beanstandete Teile aufgeteilt sein kann. Wichtig ist immer, daß eine ausreichende Verschwenkbarkeit der Streifen zueinander ermöglicht wird und daß die Streifen in Quer- bzw. Tangentialrichtung einen ausreichenden Zusammenhalt haben.

Dabei können die Streifen aus vollem Material oder als aus der Rolladentechnik bekannte Kasten-Hohlform, mit Innenversteifungsrippen, gefertigt sein. Bei letzterer Ausführung mit innenversteifter Kasten-Hohlform, ist die Gelenkachse in Art eines Hakens ausgebildet, der in die schmalseitige Gelenkpfanne des benachbarten Streifens verschwenkbar, in Längsrichtung verschiebbar (durch bekannte Mittel nach Zusammensetzen längsverschieblich arretierbar) und zudem in Tangential- bzw. Querrichtung mit etwas Spiel leicht ineinander verschiebbar, eingreift. Hierdurch wird, abgesehen von einer relativ günstigen Herstellungsweise im Falle einer Kunststoffmaterialfertigung, z. B. durch Extrudieren, auch noch der sehr große Vorteil erreicht, daß nach bereits angelegter Rollschale durch die Bänder die Schale insgesamt noch etwas enger gezogen werden kann, da sich die einzelnen Streifen in den Gelenken etwas näher zusammenschieben oder weiter auseinanderziehen lassen. Wird nach Anlegen und Festlegen der Schale festgestellt, daß sie etwas zu locker sitzt, muß somit nicht die ganze Schale abgenommen, sondern nur die Bänder etwas, z.B. bis zum nächstliegenden Klett-Festpunkt freigelegt und anschließend etwas fester über den Umfang gelegt werden, wodurch die Streifen näher zusammenrücken und der Umfang insgesamt verringert wird und so die Rollschale einen festeren Sitz erhält. Bei zu festem, klemmendem Sitz, durch welchen die Blutzirkulation im Arm unterbunden werden kann, kann leicht und schnell durch teilweises Lösen und anschließend lockerere Zuschlingen der Bänder Abhilfe geschaffen werden, ohne daß der Patient besondere weitere Hilfe benötigt oder gar die Orthese neu anlegen muß.

In weiterer Lösung der Aufgabe (gemäß Anspruch 5) ist die aus einer in Umfangsrichtung rollflexiblen Schale gebildete Orthese aus mehreren in Längserstreckung parallel zueinander angeordneten, die jeweils benachbart schwenkbar miteinander verbundenen biegesteifen Streifen zusammengesetzt und wird über Klettverschluß-bänder zusammengehalten. Dabei ist lediglich an der Innenseite der Streifen ein atmungaktives, hautfreudndliches Material wie Baumwollgewebe z.B. durch Kleben aufgebracht und die Klettverschluß-Bänder sind komplett über den äußeren Umfang der überlappend angelegten Schale immer in gleicher Anlege- oder Zugrichtung, hinweggeführt. Zudem sind die Streifen über elastische Stege miteinander verbunden und die Biegestege zwischen den Streifen sind als nach außen geschweifte Bögen ausgebildet, wobei die Streifen eine den Bögen angenäherte Dicke aufweisen, wodurch eine annähernd wellblechähnliche Ausbildung gegeben ist, insgesamt ein in Querrichtung flexibles und in Längserstreckung steifes Ganzteil-Element bildend.

Bei dieser Ausführungsform mit direkter Verbindung zwischen den Streifen ist an der Innenseite der Stege ein atmungsaktives und hautverträgliches Innenfutter, vorzugsweise aus Textilmaterial, aufzubringen ist. Auch sollten in den Stegen oder Verbindungsteilen usw. jeweils Löcher und/oder Schlitze zur ausreichendes Belüftung vorgesehen sein. Durch die vom Anlegeumfang abgehobenen Stege und zusätzlich angeordneten Schlitze oder Löcher, kann so eine gute Luftzirkulation durch die unter den Stegen gebildeten Kanal-Hohlräume erfolgen.

Von Vorteil ist, wenn die Streifen der Rollschale aus Kunststoff, Holz oder Metall oder einer Kombination dieser Materialien gefertigt wird. Die Materialwahl kann individuell erfolgen, je nach den Kriterien, auf die Wert gelegt wird, wie Preis, Umweltbewußtsein usw.

Die Streifen sind vorzugsweise aus Hart-Material, z.B. HartKunststoff gefertigt und mit einer Breite von ca. 9 mm und einer Dicke von ca. 3 mm ausgelegt.

Von ganz besonderem Vorteil ist, wenn bei der Ausbildung der Orthese als Humerus-Fraktur-Orthese eine im wesentlichen L-förmige, bzw. dem Arm-Schulter-Verlauf angepaßte Schulterbegrenzungskappe an im angelegtem Zustand äußeren Abschnitt der Rollschale positionsverstellbar befestigt ist. Diese Schulterbegrenzungskappe ist in der oberen Schalenzone im wesentlichen mittig so befestigt, daß sie dem äußeren Verlauf des Armes folgend zuerst im wesentlichen vertikal und danach über der Schulter hin kappenartig verläuft bzw. ausläuft, sich mit dem oberen Abschnitt auf der Schulter im wesentlichen vertikal abstützend. Hierdurch wird ein Nachuntenrutschen der Orthese verhindert, da stets eine vertikale Abstützung vorhanden ist. Auch ist zusätzlich eine Kontrolle über die Bewegung des Oberarmes durch den Patienten vorhanden.

Zwar ist bekannt, an Orthesen Begrenzungskappen bzw. -elemente vorzusehen, um einer Längsverschieblichkeit der Orthese zu begegnen. Diese sind jedoch nach vorhergehendem Anpassen an die entsprechende Längsposition zueinander definitiv festgelegt, so daß ein nachträgliches, bedarfsweises Längsverschieben oder Verschwenken der Kappe nicht mehr möglich ist. Der Patient muß mit der einmal vorgenommenen Positionierung der Orthese auskommen, auch wenn diese nicht optimal ist.

Die erfindungsgemäße Schulterbegrenzungskappe weist in vorteilhafter Weise einen langen, vertikalen Führungsarm und einen kurzen, etwa horizontalen Abstützarm auf, die bogenförmig ineinander übergehen. Der Abstützarm weist dabei an seinem äußeren Ende unterseitig eine Polsterung auf, die als runder Schaumplast-Polster, oder als über die gesamte Schulterrundung hinwegführende Längsstreifen-Polsterung ausgebildet ist. Die runde Polsterung ist vorzuziehen, da durch diese eine genauere Vertikalabstützung erzielt wird.

Der längere Führungsarm der Schulterbegrenzungskappe ist mit seinem unteren Endabschnitt überlappend und formangepaßt auf der Schale aufliegend längsverschieblich und zur Vertikalen/Längserstreckung verschwenkbar befestigt. Hierdurch kann, je nach Bedarf, eine Voreinstellung oder eine Nachjustierung der Position der Orthese zur Schulter vorgenommen werden. Auch kann durch die leichte Verschwenkbarkeit der Schulterbegrenzungskappe eine optimale Aufstützung auf der Schulter erzielt werden, da die Position der Schulter von Person zu Person unterschiedlich ist, je nach deren Haltung, also je nach geradem oder mehr oder weniger gekrümmtem Rücken oder Schultern.

Die Verschieb- und/oder Verschwenkbarkeit der Schulterbegrenzungskappe über ihren Führungsarm auf der Schale wird in vorteilhafter Weise dadurch erzielt, daß am unteren, die Schale überlappenden Endabschnitt des Führungsarmes zwei zueinander parallele Längs-Führungsschlitze vorgesehen sind, durch die jeweils ein Schraubbolzen, der in der Schale fest verankert ist, senkrecht hindurchreicht. Über darauf angeordnete Flügelmuttern oder Rändelmuttern, also Muttern, die ohne Werkzeuge von Hand schnell und ohne besondere Kraftanstrengung betätigt werden können, kann schnell die Verbindung gelöst oder festgesetzt werden. Der Patient kann somit selbst, nach erfolgtem Anlegen der Orthese, nur durch leichtes Lockern der Schraubverbindungen die Schulterbegrenzungskappe in Längsrichtung verschieben und/oder aus der Vertikalposition weiter nach vorne oder hinten leicht verschwenken, so daß eine optimale Abstützung und ein sehr guter Tragekomfort erhalten wird, bei optimalen Sitz- und Trageeigenschaften der Orthese.

Von Vorteil ist des weiteren, wenn die Schulterbegrenzungskappe rinnenförmig, mit im wesentlichen gleichem Krümmungsradius geformt ist, wie die Schale in angelegtem Zustand oder leicht querelastisch eine tangentiale Schwenkverformung beim An- und Ablegen der Orthese erlaubt und wenn sie aus im wesentlichen gleichem Material gefertigt ist wie die Streifen der Rollschale und vorzugsweise aus mit Luftlöchern versehenem wasserfesten Kunststoff besteht.

Eine angenehae Trageeigenschaft wird erzielt, wenn das obere Ende des Abstützarmes der Schulterbegrenzungskappe halbkreisförmig abgerundet ist, gleichzeitig der runden Polsterung teilweise konturmäßig folgend.

Nachfolgend wird die Erfindung anhand mehrerer Ausfuhrungsbeispiele unter Bezug auf die Zeichnung näher erläutert.

Es zeigt:
- Fig. 1:: eine Seitenansicht auf eine angelegte Roll-Orthese mit Schulterbegrenzungskappe,
- Fig. 2:: eine perspektivische Vorderansicht auf eine Orthese nach Fig. 1,
- Fig. 3:: eine Rückansicht auf die Orthese gemäß Fig. 1 und 2,
- Fig. 4:: eine Draufsicht auf eine Orthese gemäß Fig. 1 bis 3, in abgelegter, flach aufgerollter Form, in Ausführung für den rechten Arm,
- Fig. 5:: einen teilweisen Schnitt nach den Linien V - V aus Fig. 4, in weiterer Ausführung (Rolladesausführung),
- Fig. 6:: eine teilweise Draufsicht in teilweisem Schnitt nach dem Pfeil VI aus Fig. 5,
- Fig. 7:: eine Längsschnialseitenansicht auf eines RollschalenStreifen gemäß Pfeil VII aus Fig. 6,
- Fig. 8:: eine teilweise Ansicht in Längsverlaufrichtung auf eine Roll-Schale in weiterer Ausführung,
- Fig. 9:: eine Draufsicht gemäß Pfeil IX aus Fig. 8,
- Fig. 10:: eine Teilansicht in Längsverlaufrichtung auf eine Roll-Schale mit bogenförmigen Stegen, und
- Fig. 11:: eine Draufsicht auf die Roll-Schale nach Fig. 10.

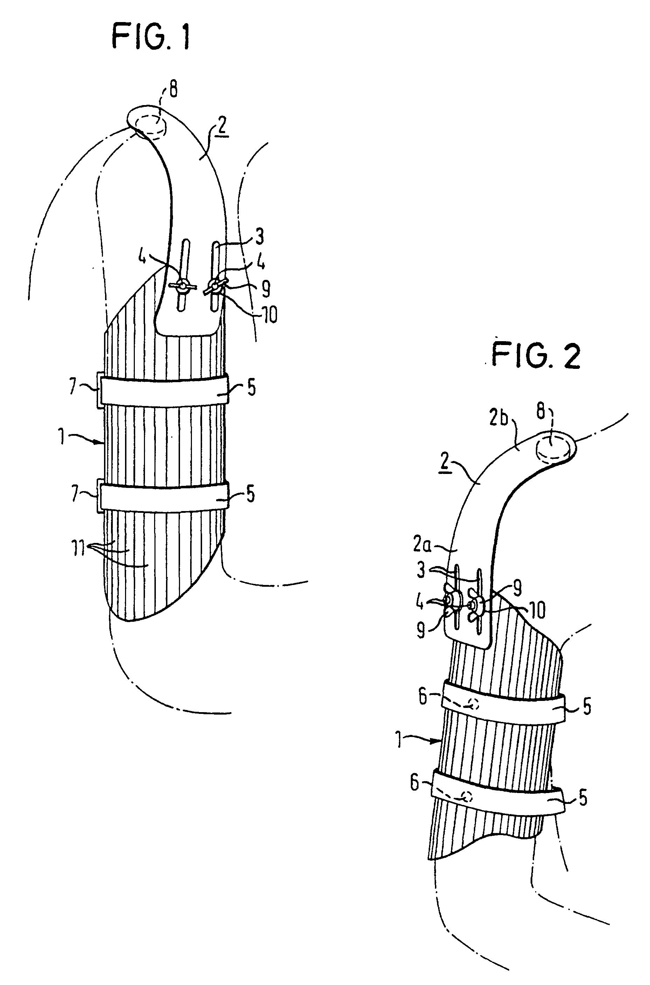

Wie aus Fig. 1 bis 4 hervorgeht, die die erfinduagsgemäße Orthese in einer Ausführung als Humerus-Fraktur-Orthese zeigen, besteht diese Orthese aus einer einzigen, rollflexiblen Schale 1 (Roll-Schale), die überlappend an dem Oberarm angelegt ist und in diesem Zustand über zwei Klettverschluß-Bänder 5 festgehalten wird. Die Klettverschluß-Bänder sind dabei an der Roll-Schale 1 über Klett-Befestigungspunkte 6 und Kunststoffoder Metall-Führungsschlaufen 7 positionsfestgelegt. Die Bänder 5 verlaufen dabei stets in gleicher Umfangsrichtung um die angelegte Roll-Schale herum und sind mit ihrem äußeren Ende auf einem entsprechenden oberen Klettpunkt des Baadanfangs festgelegt.

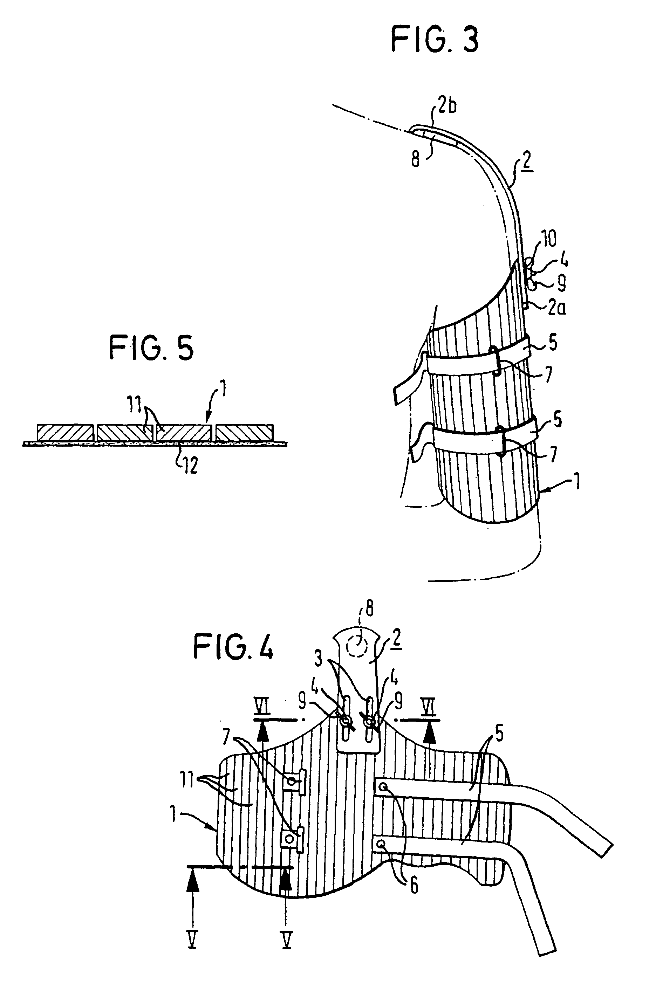

Wie insbesondere aus Fig. 4 zu erkennen ist, die eine Ausführung der Orthese für einen rechten Arm der Orthese darstellt, ist die Roll-Schale 1 mit verschiedenen Ausschnitten an der oberen und unteren Kante versehen, die einen optimalen Sitz in bezug auf Achsel und Ellbogen erlaubt. Die Klettbänder sind an der einen Seite bzw. Hälfte der Roll-Schale 1 befestigt und weisen an der Oberseite ihres einen, mit der Roll-Schale fest verbundenen Endes jeweils einen Klett-Befestigungspunkt 6 auf. Auf der anderen Schalenhälfte sind Führungsschlaufen 7 vorgesehen, durch die nach Anlegen der Schale 1 die Bänder 5 positionsfixiert hindurchgeführt werden.

Wie aus Fig. 1 bis 4, inbesondere aus Fig. 4 zu erkennen ist, ist an der oberen Zone der Roll-Schale 1 und zwar im wesentlichen mittig, eine Schulterbegrenzungskappe 2 befestigt, die im wesentlichen L-förmig ausgebildet ist, annähernd der Schulter-Arm-Form bzw. -Krümmung folgend. Sie besteht aus einer in angelegtem Zustand ungefähr vertikal verlaufenden Führungsarm 2a und einem oberen, in die Horizontale auslaufenden Abstützarm 2b, die durch einen zwischenliegenden Krümmungsteil ineinander übergehen.

Am unteren, die hintere Schale überlappenden Ende des Führungsarms 2a sind zwei zueinander parallele, vertikal bzw. in Längsrichtung weisende Führungsschlitze 3 eingebracht, durch die jeweils ein in der Schale 1 senkrecht herausragend befestigter Schraubbolzen 4 hindurchragt. Auf diesen Schraubbolzen 4 sitzen jeweils Flügelmuttern 9, unter die Unterlegscheiben 10 gelegt sind, wie genauer aus Fig. 5 zu entnehmen ist, um eine gute Abstützung der Muttern zu ermöglichen.

Am oberen, abgerundeten Ende des Abstützarmes 2b ist unterseitig eine runde Polsterung, z.B. aus Schaumstoff oder als Schaumstoffgefüllter Polster 8, ausgebildet, angeordnet. Hierdurch wird eine quasi-punktuelle Vertikalabstützung der Orthese in angenehmer Weise sichergestellt.

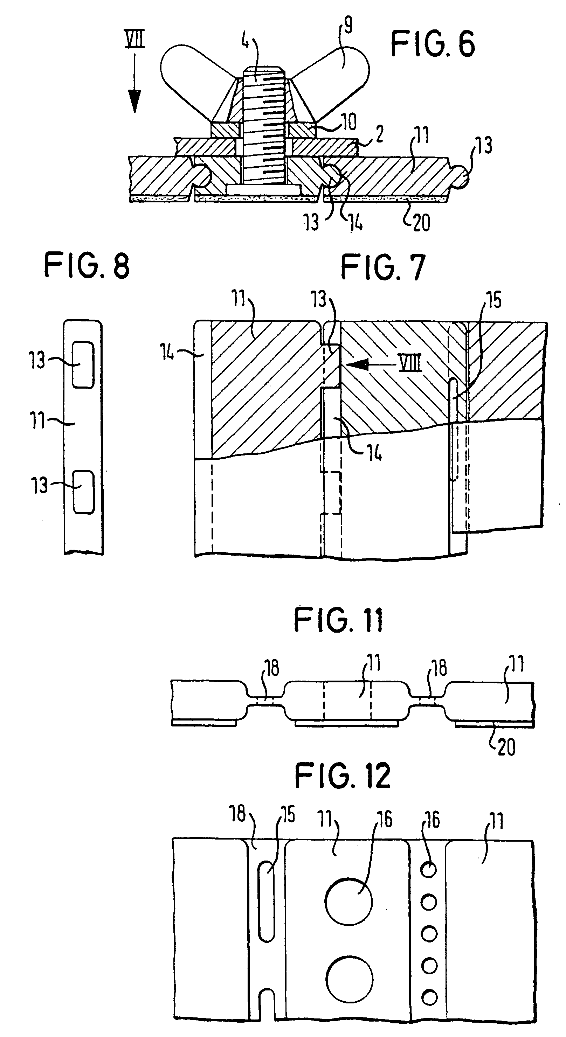

Bei den in Fig. 5 bis 7 dargestellten Ausführungsbeispielen sind leistenförmige, biegesteife nahe nebeneinander, parallel angeordnete Streifen 11 direkt miteinander gelenkig verbunden, wobei an der einen Längsschmalseite Gelenkachsen 13 vorgesehen sind, die in an der anderen Längsschmalseite angeordnete Gelenkpfannen 14 bzw. Lager eingreifen. Die Achsen 13 und Lager bzw. Pfannen 14 sind in dieser Ausführungsform im wesentlichen zylindrisch ausgebildet und können, wie aus Fig. 6 und 7 ersichtlich, durchgehend einstückig oder mehrfach unterteilt ausgebildet sein. An der Innenseite der jeweiligen Streifen 11 ist jeweils ein atmungsaktives, hautfreundliches Innenfutter 20 aufgebracht, z. B. durch Kleben.

Aus Fig. 5 ist zu entnehmen, wie z.B. die Schulterbegrenzungskappe 2 an den Streifen 11 der Roll-Schale 1 über Schraubbolzen 4 und Muttern 9, hier Flügelmuttern, mit untergelegten Beilagscheiben 10, befestigt ist.

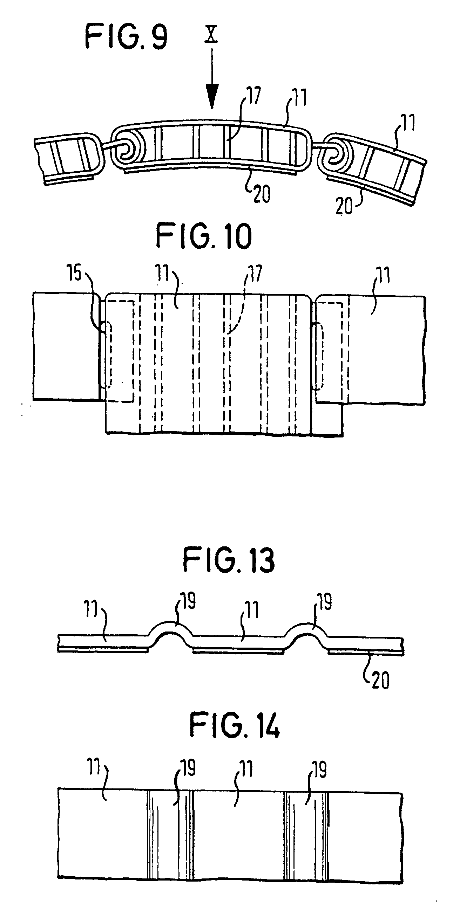

Bei dem Ausführungsbeispiel nach Fig. 8 und 9 sind die Streifen 11 als Hohl-Kastenform, mit Innenverstärkungsrippen 17 ausgebildet. Die Gelenkachsen 13 sind als Haken ausgebildet, die in Lageröffnungen bzw. Gelenkpfannen 14 so eingreifen, daß diese den schmalen Befestigungs-Steg des Hakens eng umschließen, wobei die Hakenlänge im Verhältnis zur Gelenkpfannentiefe so ausgebildet ist, daß ein zumindest geringfügige Tangential- bzw. Querein- bzw. -ausschieben der Streifen über ihre Schwenkverbindung möglich ist. Aus Fig. 8 ist zu erkennen, daß die Streifen 11 eine dem Umfang angepaßte Krümmung aufweisen können. Fig. 9 zeigt, daß in der Haken-Basis Belüftungs-Schlitze 15 vorgesehen sind, die eine gute Luftzirkulation sicherstellen.

Schließlich zeigen Fig. 10 und 11 ein Ausführungsbeispiel, bei dem zwischen den Streifen 11 biegeelastische Stege vorgesehen sind, die als nach außen geschwungene Bögen 19 ausgebildet sind. Diese Bögen 19 erlauben insbesondere eine Flexibilität in Umfangsrichtung und ggf. ein Rollen nach innen und verstärken die Längssteifigkeit, in Art eines Wellblechs oder einer Wellpappe.

In allen Ausführungsbeispielen nach Fig. 5 bis 11 ist zu erkennen, daß jeweils an der Innenseite der Schale 1 an den Streifen 11 ein atmungsaktives Innenfutter 20 aufgebracht ist, z.B. durch Kleben.

Abschließend sei darauf hingewiesen, daß die erfindungsgemäße Roll-Orthese in wesensgleicher Ausführung, jedoch ohne Schulterbegrenzungskappe als Ulna-Fraktur-Orthese (Unterarm-Orthese), und mit einer Fersenkappe, statt der Schulterbegrenzungskappe, als Tibia-Fraktur-Orthese (Bein-Orthese) anwendbar ist, nur jeweils mit leicht abgeänderter Konturgebung (distal und proximal) der Roll-Schale.

### Bezugszeichenliste

- 1: (Roll-)Schale
- 2: Schulterbegrenzungskappe
- 2a: Führungsarm
- 2b: Abstützarm
- 3: Führungsschlitze
- 4: Muttern
- 5: Klettverschluß-Bänder
- 6: Befestigungspunkte
- 7: Führungschlaufen
- 8: Polster
- 9: Schraubbolzen
- 10: Unterlegscheibe
- 11: Streifen
- 12 13: Gelenkachse
- 14: Gelenkpfanne
- 15: Schlitze
- 16: Löcher
- 17: Rippen
- 18: Stege
- 19: Bögen
- 20: Innenfutter

## Patentansprüche

1. Fraktur-Orthese, insbesondere Humerus-Fraktur-Orthese, mit einer die abzustützenden Gliedmaßen umgebenden, in Umfangsrichtung rollflexiblen Schale (1), die in Längserstrekkung aus parallel angeordneten, jeweils benachbart schwenkbar miteinander verbundenen biegesteifen Streifen (11) zusammengesetzt ist, **dadurch gekennzeichnet, daß** die Schale über Klettverschluß-Bänder zusammengehalten wird, wobei lediglich an der Innenseite der Streifen (11) ein atmungsaktives, hautfreundliches Material (20) aufgebracht ist und die Klettverschluß-Bänder (5) komplett um den äußeren Umfang der überlappend angelegten Schale (1), immer in gleicher Anlege- oder Zugrichtung hinweggeführt sind,
und wobei die Streifen (11) untereinander direkt gelenkig verbunden sind, wobei jeder Streifen (11) an seiner einen Längs-Schmalseite eine seitlich herausragende, zumindest teilweise zylindrische, in Längsrichtung sich erstreckende Gelenkachse (13) aufweist, die schwenkbar in eine sich in Längsrichtung erstreckende zylindrische Gelenkpfanne (14) oder Lager der anderen Längs-Schmalseite eines benachbarten Streifens (11) längsverschiebbar und verschwenkbar, jedoch in Umfangsrichtung nicht herausziehbar, hereinragt.

2. Orthese nach den Anspruch 1,
**dadurch gekennzeichnet, daß** die Gelenkachse (13) in Längsrichtung einstückig mit Längs-Lüftungsschlitzen (15) oder Löchern (16) versehen ausgebildet sind, oder aus mehreren, zueinander in Längsrichtung beabstandeten Gelenkachsstücken besteht.

3. Orthese nach Anspruch 1,
**dadurch gekennzeichnet, daß** die Streifen (11) aus vollem Material oder in Kasten-Hohlform, mit Innenversteifungsrippen (17) gefertigt sind.

4. Orthese nach den Ansprüchen 1 bis 3,
**dadurch gekennzeichnet, daß** die Streifen (11) als Rolladenelemente ausgebildet sind, wobei eine schmalseitig herausragende Gelenkachse (13), die als Gelenkhaken ausgebildet ist, des einen Streifens (11) in die schmalseitige Gelenkpfanne (14) des benachbarten Streifens (11) schwenkbar, in Längsrichtung verschiebbar und zudem in Tangential- bzw. Umfangsrichtung der Gelenkpfanne (14) mit etwas Spiel leicht ineinander verschiebbar eingreift.

5. Fraktur-Orthese, insbesondere Humerus-Fraktur-Orthese, mit einer die abzustützenden Gliedmaßen umgebenden, in Umfangsrichtung rollflexiblen Schale (1), die in Längserstrekkung aus parallel angeordneten, jeweils benachbart schwenkbar miteinander verbundenen biegesteifen Streifen (11) zusammengesetzt ist, **dadurch gekennzeichnet, daß** die Schale über Klettverschluß-Bänder zusammengehalten wird, wobei lediglich an der Innenseite der Streifen (11) ein atmungsaktives, hautfreundliches Material (20) aufgebracht ist und die Klettverschluß-Bänder (5) komplett um den äußeren Umfang der überlappend angelegten Schale (1), immer in gleicher Anlege- oder Zugrichtung hinweggeführt sind, und wobei die Streifen (11) über elastische Stege (19) miteinander verbunden sind und die Stege zwischen den Streifen (11) als nach außen geschweifte Bögen (19) ausgebildet sind, wobei die Streifen (11) eine den Bögen (19) angenäherte Dicke aufweisen, wobei eine annähernd wellblechähnliche Ausbildung gegeben ist, insgesamt ein in Querrichtung flexibles und in Längserstreckung steifes Ganzteil-Element bildend.

6. Orthese nach Anspruch 1 und 5,
**dadurch gekennzeichnet, daß** die Streifen (11) aus Kunststoff, Holz oder Metall oder einer Kombination dieser Materialen gefertigt sind und vorzugsweise ca. 9 mm breit und ca. 3 mm dick sind.

7. Orthese nach Anspruch 1 und 5,
**dadurch gekennzeichnet, daß** bei der Ausbildung der Orthese als Humerus-Fraktur-Orthese eine L-förmige oder dem Arm-Schulterverlauf angepaßte Schulterbegrenzungskappe (2) vorgesehen ist, die in angelegtem Zustand am äußern Abschnitt der Rollschale (1) von Hand positionseinstellbar befestigt ist.

8. Orthese nach Anspruch 7,
**dadurch gekennzeichnet, daß** die Schulterbegrenzungskappe (2), in angelegtem Zustand gesehen, einen langen, vertikalen Führungsarm (2a) und einen kurzen, horizontal weisenden Abstützarm (2b) aufweist, die bogenförmig ineinander übergehen.

9. Ortbese nach den Ansprüchen 7 und 8,
**dadurch gekennzeichnet, daß** der Abstützarm (2b) an der Unterseite seines äußeren Endes eine Polsterung (8) aufweist, die kreisförmig ausgebildet ist oder als Längsstreifen über die gesamte Schulterrundung hinweggeführt sein kann.

10. Orthese nach den Ansprüchen 7 bis 9,
**dadurch gekennzeichnet, daß** der Führungsarm (2a) mit seinem unteren Endabschnitt überlappend und formangepaßt auf der Schale (1) aufliegend längsverschieblich und gleichzeitig geringfügig zur Vertikalen/Längserstreckung verschwenkbar befestigt ist.

11. Orthese nach Anspruch 10,
**dadurch gekennzeichnet, daß** am unteren Endabscbnitt des Führungsarmes (2a) zwei zueinander parallele Längs-Führungsschlitze (3) vorgesehen sind, durch welche jeweils ein in der Schale (1) senkrecht herausragend befestigter Schraubbolzen (9) so hindurchreicht, daß jeweils eine Mutter (4), wie eine Flügelmutter oder Rändelmutter, an den herausragenden Enden der Schraubbolzen (4) festschraubbar sind.

12. Orthese nach den Ansprüchen 7 bis 11,
**dadurch gekennzeichnet, daß** die Schulterbegrenzungskappe (2) rinnenförmig, mit gleichen Krümmungsradius geformt ist, wie die Schale (1) in angelegtem Zustand, oder leicht querelastisch eine Schwenkverformung beim An- und Ablegen der Orthese erlaubt und daß sie aus gleichem Material gefertigt ist, wie die Streifen (11) der Rollschale (1), wie aus mit Luftlöchern versehenem, wasserfestem Kunststoff.

13. Orthese nach den Ansprüchen 7 bis 11,
**dadurch gekennzeichnet, daß** die Rollschale (1), deren Streifen (11) und auch die Kappe (2) an ihren Innenseiten mit einem atmungsaktiven, hautverträglichen Innenfutter (20) versehen sind, wobei dieses Innenfutter eine textile, Baurwollgewebebespannung oder Plüschauskleidung sein kann.

## Claims

1. Fracture orthesis, in particular humerus fracture orthesis, with a shell (1) with rolling flexibility in the circumferential direction and which surrounds the limb masses to be supported and in the longitudinal extension is composed of flexurally stiff strips (11) which are arranged parallel and in each case connected to one another so as to be pivotable with respect to their neighbours, **characterised in that** the shell is held together by means of hook and loop fastening tapes, and a breathable skin-friendly material (20) is applied only on the inside of the strips (11) and the hook and loop fastening tapes (5) are passed completely around the outer circumference of the shell (1) applied so that it overlaps, always in the same direction of application or tension, and the strips (11) are hinged directly to one another, each strip (11) exhibiting on its first longitudinal narrow side a laterally projecting, at least partly cylindrical hinge pivot (13) extending in the longitudinal direction and projecting pivotably into a cylindrical hinge socket (14) or bearing extending in the longitudinal direction on the other longitudinal narrow side of a neighbouring strip (11) so that it is displaceable longitudinally and pivotable but cannot be extracted in the circumferential direction.

2. Orthesis according to claim 1, **characterised in that** in the longitudinal direction the hinge pivot (13) is formed in one piece and provided with longitudinal ventilating slots (15) or holes (16) or consists of a plurality of hinge pivot parts spaced relative to one another in the longitudinal direction.

3. Orthesis according to claim 1, **characterised in that** the strips (11) are made of solid material or in a hollow box shape with internal stiffening ribs (17).

4. Orthesis according to claims 1 to 3, **characterised in that** the strips (11) are embodied as roller blind elements, and a hinge pivot (13) projecting on the narrow side and embodied as a hinge hook on one strip (11) engages pivotably in the hinge socket (14) on the narrow side of the neighbouring strip (11) so that it is displaceable in the longitudinal direction and also displaceable easily with some play in the tangential or circumferential direction of the hinge socket (14).

5. Fracture orthesis, in particular humerus fracture orthesis, with a shell (1) with rolling flexibility in the circumferential direction and which surrounds the limb masses to be supported and in the longitudinal extension is composed of flexurally stiff strips (11) which are arranged parallel and in each case connected to one another so as to be pivotable with respect to their neighbours, **characterised in that** the shell is held together by means of hook and loop fastening tapes, and a breathable skin-friendly material (20) is applied only on the inside of the strips (11) and the hook and loop fastening tapes (5) are passed completely around the outer circumference of the shell (1) applied so that it overlaps, always in the same direction of application or tension, and the strips (11) are connected to one another by means of elastic links (19) and the links between the strips (11) are embodied as outwardly curved bows (19), the strips (11) exhibiting a thickness roughly matching the bows (19), producing a design roughly similar to a corrugated sheet, forming in all a whole element which is flexible in the transverse direction and stiff in the longitudinal extension.

6. Orthesis according to claim 1 and 5, **characterised in that** the strips (11) are made of plastic, wood or metal or a combination of these materials and are preferably approximately 9 mm wide and approximately 3 mm thick.

7. Orthesis according to claim 1 and 5, **characterised in that** when the orthesis is embodied as a humerus fracture orthesis, a shoulder limiting cap (2) is provided which has an L shape or is adapted to the line of the arm and shoulder and when fitted is fastened in a manually adjustable position on the outer portion of the rolling shell (1).

8. Orthesis according to claim 7, **characterised in that** the shoulder limiting cap (2), viewed when fitted, exhibits a long vertical guiding arm (2a) and a short horizontally pointing supporting arm (2b) which pass into one another in a curve.

9. Orthesis according to claims 7 and 8, **characterised in that** on the underside of its outer end the supporting arm (2b) exhibits a pad (8) which is circular in shape or can be passed as a longitudinal strip over the entire curve of the shoulder.

10. Orthesis according to claims 7 to 9, **characterised in that** the guiding arm (2a) is fastened with its lower end portion resting on the shell (1), overlapping and matching the shape, so that it is displaceable longitudinally and at the same time pivotable slightly in relation to the vertical/longitudinal extension.

11. Orthesis according to claim 10, **characterised in that** in the lower end portion of the guiding arm (2a) two parallel longitudinal guiding slots (3) are provided in each case for passage of a stud (9) fastened in the shell (1) so that it projects vertically so that in each case a nut (4), such as a wing nut or knurled nut, can be screwed firmly on to the projecting ends of the studs (9) .

12. Orthesis according to claims 7 to 11, **characterised in that** the shoulder limiting cap (2) is fluted with the same radius of curvature as the shell (1) when fitted, or has slight transverse elasticity allowing pivoting deformation when the orthesis is fitted and removed, and **in that** it is made of the same material as the strips (11) of the rolling shell (1), such as waterproof plastic provided with air holes.

13. Orthesis according to claims 7 to 11, **characterised in that** the rolling shell (1), its strips (11) and the cap (2) are provided with a breathable skin-compatible internal lining (20) on their insides, and this internal lining can be a textile, cotton fabric covering or plush lining.

## Revendications

1. Orthèse pour fracture, notamment orthèse pour fracture de l'humérus, comprenant une coquille (1) qui entoure les extrémités à soutenir, qui est souple à l'enroulement dans la direction périphérique et qui est composée, s'étendant dans la direction longitudinale, de bandes (5) parallèles, rigides à la flexion et reliées à pivotement respectivement entre bandes voisines, **caractérisée en ce que** la coquille est maintenue par des rubans de fermeture auto-agrippante, un matériau (20) bon pour la peau et permettant la respiration étant déposer seulement sur la face intérieure des bandes (11) et les rubans (5) de fermeture auto-agrippante entourant complètement, en allant toujours dans la même direction d'application ou de traction, le pourtour extérieur de la coquille (1) appliqué à recouvrement,
et les bandes (11) étant articulées entre elles directement, chaque bande (11) ayant sur l'un de ses côtés longitudinaux étroits un axe (13) d'articulation qui fait saillie latéralement, qui est cylindrique au moins en partie, qui s'étend dans la direction longitudinale et qui pénètre à pivotement, avec possibilité de coulisser longitudinalement et de pivoter mais non d'être retiré dans la direction périphérique, dans une cavité (14) d'articulation cylindrique, s'étendant dans la direction longitudinale, ou dans un palier de l'autre côté (3) étroit longitudinal d'une bande (11) voisine.

2. Orthèse suivant la revendication 1,
**caractérisée en ce que** les axes (13) d'articulation sont constitués d'une seule pièce dans la direction longitudinale avec des fentes (15) d'aération longitudinales ou munis de trous (16), ou l'axe d'articulation est constitué de plusieurs tronçons d'axe d'articulation à distance les uns des autres dans la direction longitudinale.

3. Orthèse suivant la revendication 1, **caractérisée en ce que** les bandes (11) sont en matériau plein ou sous forme creuse en caisson en ayant des nervures (17) intérieures de raidissement.

4. Orthèse suivant l'une des revendications 1 à 3, **caractérisée en ce que** les bandes (11) sont constituées en élément de volet roulant, un axe (13) d'articulation qui fait saillie du côté étroit et qui est constitué en crochet d'articulation, de l'une des bandes (11) pénétrant dans la cavité (14) d'articulation du côté étroit de la bande (11) voisine, avec possibilité de pivotement, avec possibilité de coulissement dans la direction longitudinale et, en outre, avec possibilité de coulisser facilement l'un dans l'autre avec un peu de jeu, tant la direction tangentielle ou périphérique dans la cavité (14) d'articulation.

5. Orthèse pour fracture, notamment orthèse pour fracture de l'humérus, comprenant une coquille (1) qui entoure les extrémités à soutenir, qui est souple à l'enroulement dans la direction périphérique et qui est composée, s'étendant dans la direction longitudinale, de bandes (5) parallèles, rigides à la flexion et reliées à pivotement respectivement entre bandes voisines, **caractérisée en ce que** la coquille est maintenue par des rubans de fermeture auto-agrippante, un matériau (20) bon pour la peau et permettant la respiration étant déposer seulement sur la face intérieure des bandes (11) et les rubans (5) de fermeture auto-agrippante entourant complètement, en allant toujours dans la même direction d'application ou de traction, le pourtour extérieur de la coquille (1) appliquée à recouvrement,
les bandes (11) étant reliées entre elles par des barrettes (19) élastiques, et les barrettes étant constituées entre les bandes (11) sous la forme d'arceaux (19) tournant leurs concavité vers l'extérieur, les bandes (11) ayant à peu près l'épaisseur d'un arceau, ce qui donne une constitution sensiblement analogue à une tôle ondulée en formant dans l'ensemble un élément global souple dans la direction dans la direction transversale et rigide dans l'étendue longitudinale.

6. Orthèse suivant-la revendication 1 et 5, **caractérisée en ce que** les bandes (11) sont en matière plastique, en bois ou en métal ou en une combinaison de ces matériaux, étant de préférence d'une largeur de 9 mm environ et d'une épaisseur de 3 mm environ.

7. Orthèse suivant la revendication 1 et 5, **caractérisée en ce que** si l'orthèse est constituée sous la forme d'une orthèse pour la fracture de l'humérus, il est prévu un capuchon (2) de limitation à l'épaule adapté au tracé en forme de L ou au tracé de l'épaule du bras, qui est fixé à l'état appliqué à la partie extérieure de la coquille (1) avec possibilité d'être mis en position à la main.

8. Orthèse suivant la revendication 7, **caractérisée en ce que** le capuchon (2) de limitation à l'épaule a, considéré à l'état appliqué, un bras (2a) long vertical de guidage et un bras (2b) court horizontal d'appui avec passage de l'un à l'autre sous la forme d'un arceau.

9. Orthèse suivant les revendications 7 et 8, **caractérisée en ce que** le bras (2b) d'appui a sur la face inférieure de son extrémité extérieure un rembourrage (8) qui est de forme circulaire ou qui peut passer sous la forme de bandes longitudinales sur tout l'arrondi de l'épaule.

10. Orthèse suivant les revendications 7 à 9, **caractérisée en ce que** le bras (2a) de guidage est fixé avec possibilité de pivoter à chevauchement par son tronçon d'extrémité inférieure et de manière adaptée à sa forme sur la coquille (1) avec possibilité de coulisser longitudinalement et en même temps en ayant légèrement une étendue longitudinale/verticale.

11. Orthèse suivant le revendication 10, **caractérisée en ce qu'**il est prévu sur le tronçon d'extrémité inférieure du bras (2a) de guidage deux fentes (3) de guidage longitudinales, parallèles entre elles, par lesquelles respectivement un axe (9) de vis, fixé en faisant saillie perpendiculairement à la coquille (1), peut passer de façon à ce que respectivement un écrou (4), comme un écrou à oreille ou un écrou moleté, peut être fixé à l'extrémité en saillie de l'axe (4) de la vis.

12. Orthèse suivant les revendications 7 à 11, **caractérisée en ce que** le capuchon (2) de limitation à l'épaule est en forme de goulotte de même rayon de courbure que la coquille à l'état appliqué, ou permet, par une légère élasticité transversale, une déformation articulée lorsque l'on met et lorsque l'on enlève l'orthèse et **en ce qu'**il est fabriqué en le même matériau que les bandes (11) de la coquille (1), comme en matière plastique résistant à l'eau et munie de trous d'aération.

13. Orthèse suivant les revendications 7 à 11, **caractérisé en ce que** la coquille (1), ses bandes (11) et également le capuchon (2) sont munis sur leur face intérieure d'une garniture (20) intérieure permettant la respiration et compatible avec la peau, cette gamiture intérieure pouvant être un entoilage textile en tissu de coton ou un revêtement en peluché.
